# EUROPÄISCHE PATENTSCHRIFT

(11) **EP 1 362 026 B1**
(45) Veröffentlichungstag und Bekanntmachung des Hinweises auf die Patenterteilung: **27.06.2007**
(21) Anmeldenummer: 02702294.6
(22) Anmeldetag: 22.01.2002
(51) Int. Cl.: C07C 211/65

(54) **VERFAHREN ZUR HERSTELLUNG VON BIS (TRIFLUORMETHYL)IMIDO-SALZEN**
METHOD FOR PRODUCING BIS(TRIFLUOROMETHYL)IMIDO SALTS
PROCEDES DE PREPARATION DE SELS BIS(TRIFLUOROMETHYL)IMIDO

(30) Priorität: 14.02.2001 DE 10107118
(43) Veröffentlichungstag der Anmeldung: 19.11.2003
(73) Patentinhaber: Merck Patent GmbH, 64293 Darmstadt (DE)
(72) Erfinder: HEIDER, Udo, Winchester S022 4HZ (GB); SCHMIDT, Michael, 64331 Weiterstadt (DE); SARTORI, Peter, 86919 Utting (DE); IGNATYEV, Nikolai, 47058 Duisburg (DE); KUCHERINA, Andrij, 47053 Duisburg (DE); ZINOVYEVA, Ludmila, 47051 Duisburg (DE)
(74) Vertreter: Wolff, Felix
(86) Internationale Anmeldenummer: PCT/EP2002/000582
(87) Internationale Veröffentlichungsnummer: WO 2002/064542

(56) Entgegenhaltungen:
- WO-A-00/46180

## Beschreibung

Die vorliegende Erfindung betrifft neue Verfahren zur Herstellung von Bis(trifluormethyl)imido-Salzen der allgemeinen Formel (I):

M^{a+} [(N(CF₃)₂⁻]ₐ (I)

Die Chemie des Bis(trifluormethyl)imido-Anions basiert allgemein auf der chemischen Umsetzung von Perfluoro(2-azapropen), CF₃N=CF₂, als Ausgangsmaterial (H.G. Ang and Y.C. Syn, Advances in Inorganic Chemistry and Radiochemistry, Vol. 16 (1974), S. 1-64; A. Haas, Gmelin Handbook of Inorganic Chmistry, 8th edition, Springer Verlag: Berlin, Heidelberg, New York (1991), Part 9, p. 125-153; A. Haas, Gmelin Handbook of Inorganic Chmistry, 8th edition, Springer Verlag: Berlin, Heidelberg, New York (1991), Suppl. Vol. 6, p. 196-214). Diese Verbindung kann durch Fluorierung von CCl₃N=CCl₂ mit NaF in Sulfolan bei 105 °C in einer Ausbeute von 78 % hergestellt werden (E. Klauke, H. Holtschmidt, K. Findeisen, Farbenfabriken Bayer AG, DE-A1-2101107 (1971/1972) oder durch Photolyse von CF₃N-(CF₂CFCl₂)Cl (G. Sawar, R.L. Kirchmeier und J.M. Shreeve, Inorg. Chem. 28 (1989, S. 2187-2189) in Gas bei Raumtemperatur (Siedepunkt bei -33 °C), wobei spezielle technische Apparaturen für die genannte Verbindung notwendig sind.

Das sehr reaktive Di[bis(trifluormethyl)imido]Quecksilber, Hg[N(CF₃)₂]₂ wurde erstmals durch Young und seine Mitarbeiter synthetisiert (J.A. Young, S.N. Tsoukalas und R.D. Dresdner, J. Am. Chem. Soc. 80 (1958), S. 3604-3606). Diese Verbindung ist ein gutes Reagenz zur Einführung von N(CF₃)₂-Gruppen in organische Moleküle (H.G. Ang and Y.C. Syn, siehe oben; A. Haas, Gmelin Handbook of Inorganic Chmistry, 8th edition, Springer Verlag: Berlin, Heidelberg, New York (1981), Part 9, p. 45-46), jedoch keine sehr stabile Verbindung, da sie extrem feuchtigkeitsempfindlich ist. Die Synthese von Hg[N(CF₃)₂]₂ ist schwer, zeitaufwendig und setzt spezielle technische Apparaturen sowie teure Ausgangssubstanzen voraus.

Caesium bis(trifluormethyl)imid, [Cs]⁺[N(CF₃)₂]⁻, ist eine weitere Option zur Synthese von Bis(trifluormethyl)amino-Verbindungen. Dieses Salz wird durch einfaches Einleiten von Perfluoro(2-azapropen) in eine Lösung von Caesiumfluorid in trockenem Acetonitril hergestellt (A.F. Gontar, E.G. Bykovskaja und I.L. Knunyants, IZV. Akad. Nauk SSSR, Otd. Khim, Nauk (1975), S. 2279-2282).
Der Nachteil dieser Methode liegt in der Bildung eines dimeren Produktes durch die Reaktion des Ausgangsmaterials Perfluoro(2-azapropen) mit dem bereits gebildetem Caesium-Salz. Diese Reaktion ist unvermeidbar und führt zur Bildung komplexer Produktmischungen.

N(CF₃)₂-Anionen sind jedoch gut zugänglich durch die Reaktion einiger Metallfluoride mit N,N-Bis(trifluormethyl)-perfluoralkansulfonamiden oder-acylamiden ["N(CF₃)₂-Anion Herstellung und seine Verwendung' WO 00/46180, ]. Durch dieses Verfahren ist es möglich, Na, K, Rb, Cs, Ag, Cu(II) und Hg(II) - Salze mit N(CF₃)₂-Anionen zu generieren. Die analoge Reaktion von N,N-bis(trifluormethyl)-perfluoralkan-sulfonamiden oder -acylamiden mit anderen Metallfluoriden (beispielsweise ZnF₂ und CdF₂) schreitet jedoch nur sehr langsam fort, bedingt durch die schlechte Löslichkeit dieser Fluoide in organischen Lösungsmitteln.

Aufgabe der vorliegenden Erfindung war es daher, ein verbessertes Verfahren zur Herstellung von Bis(trifluormethyl)imido-Salzen zur Verfügung zu stellen.

Die erfindungsgemäße Aufgabe wird gelöst durch ein Verfahren zur Herstellung von Bis(trifluormethyl)imido-Salzen der allgemeinen Formel (I),

[M^{a+}] [(N(CF₃)₂)⁻]ₐ (I)

worin
M^{a+} ein einwertiges oder zweiwertiges Kation und a = 1 oder 2 ist,
dadurch gekennzeichnet, daß wenigstens ein Trifluormethansulfonat der allgemeinen Formel (II),

[M^{a+}] [(OSO₂CF₃)⁻]ₐ (II)

worin
M^{a+} ein einwertiges oder zweiwertiges Kation und a = 1 oder 2 ist,
in Lösung mit Bis(trifluormethyl)imido-Rubidium umgesetzt wird und das so erhaltene Bis(trifluormethyl)imido-Salz der allgemeinen Formel (I) gegebenenfalls nach üblichen Methoden gereinigt und/oder isoliert wird.

Bevorzugt sind die erfindungsgemäßen Verfahren, bei denen M^{a+} ein Natrium-, Kalium-, Cäsium-, Kupfer oder Silber-Kation ist und a = 1 bedeutet.

Besonders bevorzugt sind die erfindungsgemäßen Verfahren, bei denen M^{a+} ein Quecksilber-, Kupfer-, Zink- oder Cadmium-Kation ist und a = 2 bedeutet.

Besonders bevorzugt sind auch die erfindungsgemäßen Verfahren, bei denen a = 1 und M^{a+} ein Kation der allgemeinen Formel (III) bedeutet,

[([R_{b}¹, R_{c}² R_{d}³ Rₑ⁴]Aₓ)_{y}Kt]⁺ (III)

worin
Kt = N, P, As, Sb, S, Se,
A = N, P, P(O), O, S, S(O), SO₂, As, As(O), Sb, Sb(O),
R¹, R², R³ und R⁴, gleich oder verschieden,
H, Halogen, substituiertes und/oder unsubstituiertes Alkyl CₙH₂ₙ₊₁, substituiertes und/oder unsubstituiertes C₁₋₁₈-Alkenyl und einer oder mehreren Doppelbindungen, substituiertes und/oder unsubstituiertes C₁₋₁₈-Alkinyl und einer oder mehreren Dreifachbindungen, substituiertes und/oder unsubstituiertes Cycloalkyl CₘH₂ₘ₋₁, ein ein- oder mehrfach substituiertes und/oder unsubstituiertes Phenyl, substituiertes und/oder unsubstituiertes Heteroaryl;
mit
n=1-18,
m=3-7,
x = 0 oder 1,
y=1-4, y=1 für x=0,
bedeuten, wobei
b, c, d, e jeweils = 0 oder 1 mit b+c+d+e ≠ 0,
A in verschieden Stellungen in R¹, R², R³ und/oder R⁴ eingeschlossen sein kann,
Kt in einem cyclischen oder heterozyklischen Ring eingeschlossen sein kann,
die an Kt gebundenen Gruppen gleich oder verschieden sein können.

Mit den erfindungsgemäßen Verfahren können auch schwer zugängliche neue Bis(trifluormethyl)imido-Salze wie beispielsweise Cadmium-, Zink- oder Kupfer(I)-N(CF₃)₂-Salze hergestellt werden. Daher stellen die neuen Salze einen weiteren Gegenstand der vorliegenden Erfindung dar.

Die erfindungsgemäß hergestellten Salze können allein oder in Mischungen mit anderen Salzen als Leitsalze oder Additive in Elektrolyten verwendet werden. Die Elektrolyte enthalten neben dem Salz bzw. den Salzmischungen auch Lösungsmittel bzw. Lösungsmittelgemische.

Diese Elektrolyte werden in elektrochemischen Zellen (wie z.B. primäre und sekundäre Batterien) eingesetzt. Bevorzugt werden sie in Kondensatoren und Superkondensatoren eingesetzt.

Die Ausgangsmaterialien Bis(trifluormethyl)imido-Rubidium und die Trifluormethansulfonatsalze sind beide gut in einer Reihe von organischen Lösungsmitteln löslich. Zudem sind Metalltriflate von einer Reihe von Firmen kommerziell erhältlich.

Die Umsetzung von Rb[N(CF₃)₂] und Metalltriflaten bei Raum- der kühleren Temperaturaturen findet schnell statt, beispielsweise entsprechend des folgenden allgemeinen Reaktionsschemas:

x Rb[N(CF₃)₂] + M(OSO₂CF₃)ₓ → M [N(CF₃)₂]ₓ + x Rb(OSO₂CF₃)

Bei den erfindungsgemäßen Verfahren erfolgt die Umsetzung zu einem Bis(trifluormethyl)imido-Salz der allgemeinen Formel (I) vorzugsweise bei einer Temperatur von -60 bis +60 °C, besonders bevorzugt von -50 bis +50 °C, ganz besonders bevorzugt bei -45 bis +30 °C.

Bevorzugte Lösungsmittel für die Umsetzung zu einem Bis(trifluormethyl)-imido-Salz der allgemeinen Formel (I) sind organische Lösungsmittel, besonders bevorzugt polare organische Lösungsmittel.

Ganz besonders bevorzugte Lösungsmittel zur Umsetzung zu einem Bis(trifluormethyl)-imido-Salz der allgemeinen Formel (I) sind die Lösungsmittel Acetonitril, Benzonitril, Dimethoxyethan und/oder Propionitril oder eine Mischung von Acetonitril, Benzonitril, Dimethoxyethan und/oder Propionitril.

Bevorzugte erfindungsgemäße Verfahren enthalten ≤ 0,1 Gew.-% Wasser, vorzugsweise ≤ 0,01 Gew.-% Wasser, besonders bevorzugt ≤ 0,005 Gew.-% Wasser.

In den bevorzugten erfindungsgemäßen Verfahren wird das Trifluormethansulfonatsalz der allgemeinen Formel (II) oder das Bis(trifluormethyl)imido-Rubidium in einem molaren Überschuß ≤ 3 % oder besonders bevorzugt in äquimolaren Mengen eingesetzt.

Das durch das erfindungsgemäße Verfahren entstehende Rubidiumtriflat hat eine eingeschränkte Löslichkeit in organischen Lösungsmitteln und kann bei niedrigen Temperaturen als Feststoff vom Reaktionsgemisch abgetrennt werden.

Daher sind die erfindungsgemäßen Verfahren bevorzugt, bei denen das Bis(trifluormethyl)imido-Salz der allgemeinen Formel (I) durch Filtration bei einer Temperatur von -90 bis +30 °C, besonders bevorzugt bei -70 bis +20 °C und nach Entfernung des Lösungsmittels gereinigt wird.

Die weitere Aufreinigungsmöglichkeit von einem Bis(trifluormethyl)imido-Salz der allgemeinen Formel (I) ist durch Extraktion mit Dichlormethan und/oder Hexan und/oder Diethylether möglich. Die Extraktion mit Dichlormethan ist eine bevorzugte Ausführungsvariante der vorliegenden Erfindung.

Im folgenden wird die Erfindung anhand von Beispielen erläutert. Diese Beispiele dienen lediglich der Erläuterung der Erfindung und schränken den allgemeinen Erfindungsgedanken nicht ein.

### Beispiele

### Beispiel 1:

### Synthese von Bis(Trifluormethyl)imido - Silber-Salz

Eine aus 0,083 g (0,79 mmol) Rubidiumfluorid und 0,227 g (0,79 mmol) CF₃SO₂N(CF₃)₂ in 3,2 ml trockenem Acetonitril hergestellte Lösung von Rb[N(CF₃)₂]-Salz wurde zu einer auf -20°C gekühlten Lösung aus 0,205 g (0,79 mmol) AgOSO₂CF₃ in 1,8 ml trockenem Acetonitril unter Rühren hinzugefügt. Das Gemisch wurde eine Stunde bei -20°C gerührt. Dabei bildete sich ein weißer Bodensatz. Das Lösungsmittel Acetonitril wurde bei -20°C abgesaugt und 4 ml trockenes Dichlormethan zum Rückstand gegeben. Nach zehn Minuten Rühren bei -20°C wurde die Lösung vom Rückstand getrennt und das Lösungsmittel bei -20°C abgesaugt. Es wurden 0,149 g Ag[N(CF₃)₂]·CH₃CN als weiße kristalline Substanz gewonnen, wie durch Analyse nachgewiesen wurde. Die Ausbeute betrug 62,3 %.

Analyse (Amperometrische Titration):

| | |
|---|---|
| Ausbeute: | 35,76 % (Ag⁺); |
| Theoriewert für Ag[N(CF₃)₂]·CH₃CN: | 35,85 % (Ag⁺); |

¹⁹F NMR Spektrum (Lösungsmittel CD₂Cl₂, Referenzsubstanz CCl₃F),
   ppm: -44,56 s (CF₃)
¹H NMR Spektrum (Lösungsmittel CD₂Cl₂, Referenzsubstanz TMS),
   ppm: 2,08 s (CH₃CN)
¹⁰⁹Ag NMR Spektrum (Lösungsmittel CD₂Cl₂, Referenzpunkt: chemischer Shift von 1 M AgNO₃ in D₂O auf 0 gesetzt),
   ppm: 316,23 s, Ag

### Beispiel 2:

### Synthese von Bis(trifluormethyl)imido - Kupfer(I)-Salz

Eine aus 0,080 g (0,766 mmol) Rubidiumfluorid und 0,218 g (0,766mmol) CF₃SO₂N(CF₃)₂ in 3,2 ml trockenem Acetonitril hergestellte Lösung von Rb[N(CF₃)₂]-Salz wurde bei Raumtemperatur zu eine Lösung aus 0,194 g (0,766 mmol) CuOSO₂CF₃·CH₃CN in 1,8 ml trockenem Acetonitril unter Rühren hinzugefügt. Das Gemisch wurde eine Stunde gerührt. Dabei bildete sich ein weißer Bodensatz. Das Lösungsmittel Acetonitril wurde bei Raumtemperatur abgesaugt und 4 ml trockenes Dichlormethan zum Rückstand gegeben. Nach zehn Minuten Rühren bei Raumtemperatur wurde die Lösung vom Rückstand getrennt und das Lösungsmittel bei Raumtemperatur abgesaugt. Es wurden 0,150 g Cu[N(CF₃)₂]·CH₃CN als weiße kristalline Substanz gewonnen und durch Analyse nachgewiesen. Die Ausbeute betrug 76,5 %.
¹⁹F NMR Spektrum (Lösungsmittel CD₂Cl₂, Referenzsubstanz CCl₃F),
   ppm: -44,79 s (CF₃)
¹H-NMR Spektrum (Lösungsmittel CD₂Cl₂, Referenzsubstanz TMS),
   ppm: 2,03 s, CH₃CN

### Beispiel 3:

### Synthese von Bis(trifluormethyl)imido - Zink-Salz

Eine aus 0,080 g (0,766 mmol) Rubidiumfluorid und 0,218 g (0,766 mmol) CF₃SO₂N(CF₃)₂ in 3,2 ml trockenem Propionitril hergestellte Lösung von Rb[N(CF₃)₂]-Salz wurde bei -45°C zu einer Lösung aus 0,155 g (0,383 mmol) Zn(OSO₂CF₃)₂·CH₃CN in 1,8 ml trockenem Propionitril unter Rühren hinzugefügt. Das Gemisch wurde eine Stunde bei -45°C gerührt. Dabei bildete sich ein weißer Bodensatz. Das Gemisch wurde dann auf -78°C gekühlt und für zwei Stunden bei dieser Temperatur ohne Rühren belassen. Das Lösungsmittel wurde abgesaugt und eine geringe Menge CD₃CN (ca. 30%) hinzugefügt und die Mischung durch ¹⁹F NMR Spektroskopie bei -45°C charakterisiert.

Das Signal bei -44,83 ppm ist dem Zn[N(CF₃)₂]₂ zuzuordnen, das mit dem Lösungsmittel koordiniert ist.
Zur Isolierung des Salzes wurde das Lösungsmittel bei -30°C abgesaugt und der restliche weiße Feststoff nach Auflösen in trockenem CD₂Cl₂ zur NMR-Spektroskopie eingesetzt. Das NMR Spekrum zeigte die Gegenwart des Propionitrils in der Kristalstruktur des Salzes Zn[N(CF₃)₂]₂·C₂H₅CN auf. Dieses Salz weist nur eine geringe Stabilität als Analysensubstanz bei Raumtemperatur auf.
¹⁹F NMR Spektrum bei -40 °C (Lösungsmittel CD₂Cl₂, Referenzsubstanz CCl₃F),
   ppm: -45,97 s (CF₃, die Position des Signals ist konzentrationsabhängig)
¹H NMR Spektrum bei -40 °C (Lösungsmittel CD₂Cl₂, Referenzsubstanz TMS),
   ppm: 1,08 t (CH₃); 2,25 q (CH₂), C₂H₅CN

### Beispiel 4:

### Synthese von Bis(trifluoromethyl)imido - Cadmium-Salz

Eine aus 0,080 g (0,766 mmol) Rubidiumfluorid und 0,218 g (0,766 mmol) CF₃SO₂N(CF₃)₂ in 3,2 ml trockenem Propionitril hergestellte Lösung von Rb[N(CF₃)₂]-Salz wurde bei -45°C zu einer Lösung aus 0,188 g (0,383 mmol) Cd(OSO₂CF₃)₂·2CH₃CN in 1,8 ml trockenem Propionitril unter Rühren hinzugefügt. Das Gemisch wurde eine Stunde bei -45°C gerührt. Dabei bildete sich ein weißer Bodensatz. Das Gemisch wurde dann auf -78°C gekühlt und für zwei Stunden bei dieser Temperatur ohne Rühren belassen. Das Lösungsmittel wurde abgesaugt und eine geringe Menge CD₃CN (ca. 30Vol.-%) hinzugefügt und die Mischung durch ¹⁹F NMR Spektroskopie bei - 40°C charakterisiert. Das Signal bei -42,53 ppm ist dem Cd[N(CF₃)₂]₂ zuzuordnen, das mit dem Lösungsmittel koordiniert ist. Bei Raumtemperatur verschiebt sich das Signal der N(CF₃)₂-Gruppe im ¹⁹F NMR Spektrum auf-45,39 ppm. Das Salz Cd[N(CF₃)₂]₂·nC₂H₅CN weist nur eine geringe Stabilität als Analysensubstanz bei Raumtemperatur auf.

### Beispiel 5:

### Elektrochemische Stabilität von [N(C₂H₅)₄][N(CF₃)₂]

In einer Messzelle mit Platinelektrode, Lithiumgegenelektrode und Lithiumreferenzelektrode wurden jeweils mehrere Zyklovoltammogramme hintereinander aufgenommen. Hierzu wurde ausgehend vom Ruhepotential das Potential zuerst mit einer Vorschubsgeschwindigkeit von 20 mV/s auf 6 V gegen Li/Li⁺ erhöht und im weiteren Verlauf zurück auf das Ruhepotential gefahren. Als Elektrolyte wurden eine Lösung von [N(C₂H₅)₄][N(CF₃)₂] in Propylencarbonat verwendet.

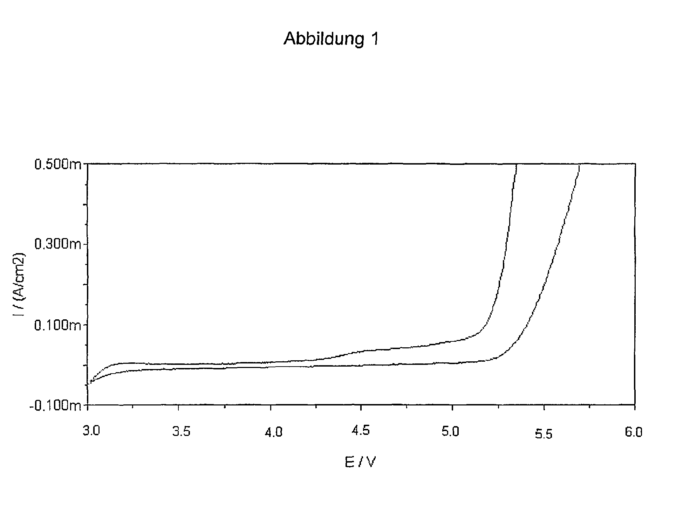

Es zeigt sich der in Abbildung 1 angegebene charakteristische Verlauf mit einem Oxidationspotential E_{Ox} größer 5V gegen Li/Li⁺.

### Beispiel 6:

### lonische Leitfähigkeit von einem [N(C₂H₅)₄][N(CF₃)₂] basierenden Elektrolyten

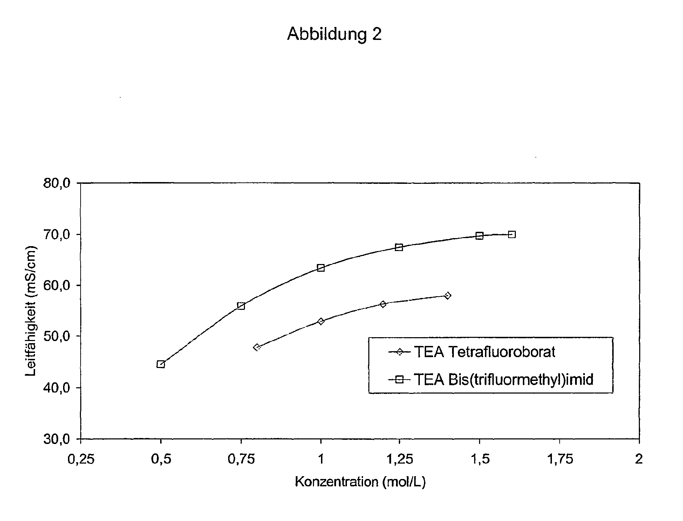

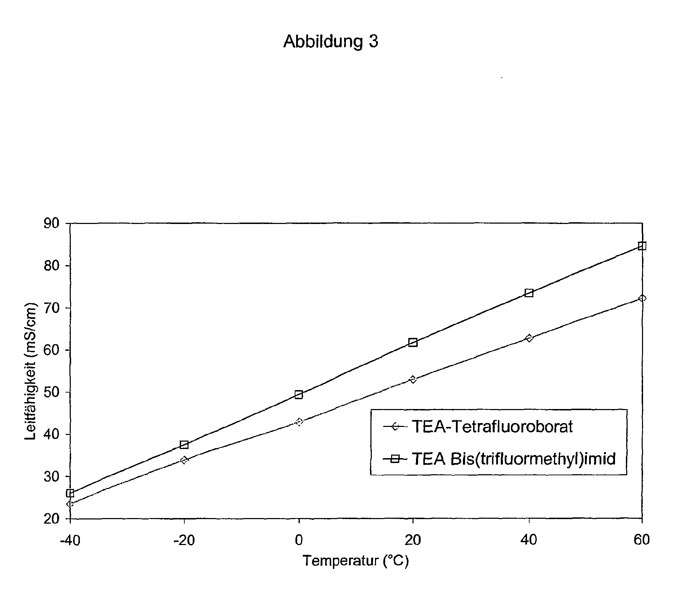

Mit Hilfe eines 4-Pol Knick-Kondutometers wurden die Leitfähigkeiten von [N(C₂H₅)₄][N(CF₃)₂] in Acetonitril in Abhängigkeit von Temperatur und Konzentration des Leitsalzes vermessen. Parallel wurde [N(C₂H₅)₄][BF₄] in Acetonitril vermessen. Dieses System stellt den derzeitigen Stand der Technik im Bezug auf "Supercapacitor"-Elektrolyte dar und dient somit als Referenz. Abbildungen 2 und 3 zeigen die erhaltenen Ergebnisse. Sie beweisen, dass das neue System auf Basis von [N(C₂H₅)₄][N(CF₃)₂] deutlich verbesserte Leitfähigkeiten aufweisen.

## Patentansprüche

1. Verfahren zur Herstellung von Bis(trifluormethyl)imido-Salzen der allgemeinen Formel (I),
M^{a+}[(N(CF₃)₂)⁻]ₐ (I)
worin
M^{a+} ein einwertiges oder zweiwertiges Kation und a = 1 oder 2 ist,
**dadurch gekennzeichnet, daß** wenigstens ein Trifluormethansulfonat der allgemeinen Formel (II),
(M^{a+}) [(OSO₂CF₃)⁻]ₐ (II)
worin M^{a+} ein einwertiges oder zweiwertiges Kation und a = 1 oder 2 ist,
in Lösung mit Bis(trifluormethyl)imido-Rubidium umgesetzt wird und das so erhaltene Bis(trifluormethyl)imido-Salz der allgemeinen Formel (I) gegebenenfalls nach üblichen Methoden gereinigt und/oder isoliert wird.

2. Verfahren gemäß Anspruch 1, **dadurch gekennzeichnet, daß** M^{a+} ein Natrium-, Kalium-, Cäsium-, Kupfer oder Silber-Kation ist und a = 1 bedeutet.

3. Verfahren gemäß Anspruch 1, **dadurch gekennzeichnet, daß** M^{a+} ein Quecksilber-, Kupfer-, Zink- oder Cadmium-Kation ist und a = 2 bedeutet.

4. Verfahren gemäß Anspruch 1, **dadurch gekennzeichnet, daß** a = 1 und M^{a+} ein Kation der allgemeinen Formel (III) bedeutet,
[([R_{b}¹, R_{c}² R_{d}³ Rₑ⁴]Aₓ)_{y}Kt]⁺ (III)
worin
Kt = N, P, As, Sb, S, Se,
A = N, P, P(O), O, S, S(O), SO₂, As, As(O), Sb, Sb(O),
R¹, R², R³ und R⁴ , gleich oder verschieden,
H, Halogen, substituiertes und/oder unsubstituiertes Alkyl CₙH₂ₙ₊₁, substituiertes und/oder unsubstituiertes C₁₋₁₈-Alkenyl und einer oder mehreren Doppelbindungen, substituiertes und/oder unsubstituiertes C₁₋₁₈-Alkinyl und einer oder mehreren Dreifachbindungen, substituiertes und/oder unsubstituiertes Cycloalkyl CₘH₂ₘ₋₁, ein ein- oder mehrfach substituiertes und/oder unsubstituiertes Phenyl, substituiertes und/oder unsubstituiertes Heteroaryl;
mit
n=1-18,
m = 3 - 7,
x = 0 oder 1,
y = 1 - 4, y = 1 für x = 0,
bedeuten, wobei
b, c, d, e jeweils = 0 oder 1 mit b+c+d+e ≠ 0,
A in verschieden Stellungen in R¹, R², R³ und/oder R⁴ eingeschlossen sein kann,
Kt in einem cyclischen oder heterozyklischen Ring eingeschlossen sein kann,
die an Kt gebundenen Gruppen gleich oder verschieden sein können.

5. Verfahren gemäß einem der Ansprüche 1 bis 4, **dadurch gekennzeichnet, daß** die Umsetzung zu einem Bis(trifluormethyl)imido-Salz der allgemeinen Formel (I) bei einer Temperatur von -60 bis +60 °C, vorzugsweise von -50 bis +50 °C, besonders bevorzugt bei -45 bis +30 °C erfolgt.

6. Verfahren gemäß einem der Ansprüche 1 bis 5, **dadurch gekennzeichnet, daß** die Umsetzung zu einem Bis(trifluormethyl)imido-Salz der allgemeinen Formel (I) in einem organischen Lösungsmittel, vorzugsweise in einem polaren organischen Lösungsmittel erfolgt.

7. Verfahren gemäß Anspruch 6, **dadurch gekennzeichnet, daß** als Lösungsmittel Acetonitril, Benzonitril, Dimethoxyethan und/oder Propionitril oder eine Mischung von Acetonitril, Benzonitril, Dimethoxyethan und/oder Propionitril verwendet wird.

8. Verfahren gemäß einem der Ansprüche 1 bis 7, **dadurch gekennzeichnet, daß** das Lösungsmittel ≤ 0,1 Gew.-%, vorzugsweise ≤ 0,01 Gew.-%, besonders bevorzugt ≤ 0,005 Gew.-% Wasser enthält.

9. Verfahren gemäß einem der Ansprüche 1 bis 8, **dadurch gekennzeichnet, daß** das Trifluormethansulfonatsalz der allgemeinen Formel (II) oder das Bis(trifluormethyl)imido-Rubidium in einem molaren Überschuß ≤ 3 % oder in äquimolaren Mengen eingesetzt werden.

10. Verfahren gemäß einem der Ansprüche 1 bis 9, **dadurch gekennzeichnet, daß** das Bis(trifluormethyl)imido-Salz der allgemeinen Formel (I) durch Filtration, vorzugsweise durch Filtration bei einer Temperatur von -90 bis +30 °C, besonders bevorzugt bei -70 bis +20 °C und nach Entfernung des Lösungsmittels gereinigt wird.

11. Verfahren gemäß einem der Ansprüche 1 bis 10, **dadurch gekennzeichnet, daß** das Bis(trifluormethyl)imido-Salz der allgemeinen Formel (I) durch Extraktion mit Dichlormethan und/oder Hexan und/oder Diethylether gereinigt wird.

12. Verbindungen der allgemeinen Formel (I),
M^{a+} [(N(CF₃)₂)⁻]ₐ (I)
worin
M = Cd²⁺, Zn²⁺oder Cu⁺
bedeutet.

13. Elektrolyt, enthaltend mindestens ein Salz der allgemeinen Formel (I), hergestellt gemäß Anspruch 1.

14. Verwendung der Elektrolyte gemäß Anspruch 13 in elektrochemischen Zellen.

15. Elektrochemische Zellen, insbesondere Kondensatoren und Superkondensatoren, enthaltend einen Elektrolyten gamäß Anspruch 13.

## Claims

1. Process for the preparation of bis(trifluoromethyl)imido salts of the general formula (I)
M^{a+}[(N(CF₃)₂)]ₐ (I)
in which
M^{a+} is a monovalent or divalent cation, and a = 1 or 2,
**characterised in that** at least one trifluoromethanesulfonate of the general formula (II)
[M^{a+}][(OSO₂CF₃)⁻]ₐ (II)
in which
M^{a+} is a monovalent or divalent cation, and a = 1 or 2,
is reacted with bis(trifluoromethyl)imidorubidium in solution, and the resultant bis(trifluoromethyl)imido salt of the general formula (I) is, if desired, purified and/or isolated by conventional methods.

2. Process according to Claim 1, **characterised in that** R^{a+} is a sodium, potassium, caesium, copper or silver cation, and a =1.

3. Process according to Claim 1, **characterised in that** M^{a+} is a mercury, copper, zinc or cadmium cation, and a = 2.

4. Process according to Claim 1, **characterised in that** a = 1 and M^{a+} is a cation of the general formula (III)
[([R_{b}¹R_{c}² R_{d}³ Rₑ⁴]Aₓ)_{y}Kt]⁺ (III)
in which
Kt = N, P, As, Sb, S or Se,
A = N, P, P(O), O, S, S(O), SO₂, As, As(O), Sb or Sb(O),
R¹, R², R³ and R⁴ are identical or different and are
H, halogen, substituted and/or unsubstituted alkyl CₙH₂ₙ₊₁, substituted and/or unsubstituted C₁₋₁₈-alkenyl having one or more double bonds, substituted and/or unsubstituted C₁₋₁₈-alkynyl having one or more triple bonds, substituted and/or unsubstituted cycloalkyl CₘH₂ₘ₋₁, monosubstituted, polysubstituted and/or unsubstituted phenyl, substituted and/or unsubstituted heteroaryl;
where
n=1-18,
m=3-7,
x=0 or 1,
y = 1 - 4, y = 1 for x = 0,
where
b, c, d and e are each = 0 or 1, where b+c+d+e ≠ 0,
A may be included in various positions in R¹, R², R³ and/or R⁴,
Kt may be included in a cyclic or heterocyclic ring,
the groups bonded to Kt may be identical or different.

5. Process according to one of Claims 1 to 4, **characterised in that** the conversion to a bis(trifluoromethyl)imido salt of the general formula (I) is carried out at a temperature of from -60 to +60°C, preferably from -50 to +50°C, particularly preferably at from -45 to +30°C.

6. Process according to one of Claims 1 to 5, **characterised in that** the conversion to a bis(trifluoromethyl)imido salt of the general formula (I) is carried out in an organic solvent, preferably in a polar organic solvent.

7. Process according to Claim 6, **characterised in that** the solvent used is acetonitrile, benzonitrile, dimethoxyethane and/or propionitrile or a mixture of acetonitrile, benzonitrile, dimethoxyethane and/or propionitrile.

8. Process according to one of Claims 1 to 7, **characterised in that** the solvent comprises ≤ 0.1% by weight, preferably ≤ 0.01% by weight, particularly preferably ≤ 0.005% by weight, of water.

9. Process according to one of Claims 1 to 8, **characterised in that** the trifluoromethanesulfonate salt of the general formula (II) or the bis(trifluoromethyl)imidorubidium is employed in a molar excess of ≤ 3% or in equimolar amounts.

10. Process according to one of Claims 1 to 9, **characterised in that** the bis(trifluoromethyl)imido salt of the general formula (I) is purified by filtration, preferably by filtration at a temperature of from -90 to +30°C, particularly preferably at from -70 to +20°C, after removal of the solvent.

11. Process according to one of Claims 1 to 10, **characterised in that** the bis(trifluoromethyl)imido salt of the general formula (I) is purified by extraction with dichloromethane and/or hexane and/or diethyl ether.

12. Compounds of the general formula (I)
M^{a+}[(N(CF₃)₂)]ₐ (I)
in which
M = Cd²⁺, Zn²⁺or Cu⁺.

13. Electrolyte comprising at least one salt of the general formula (I) prepared according to Claim 1.

14. Use of the electrolyte according to Claim 13 in electrochemical cells.

15. Electrochemical cells, in particular capacitors and supercapacitors, containing an electrolyte according to Claim 13.

## Revendications

1. Procédé pour la préparation de sels de type bis(trifluorométhyl)imido de formule générale (I),
M^{a+}[(N(CF₃)₂)⁻]ₐ (I)
où
M^{a+} représente un cation monovalent ou divalent et a = 1 ou 2,
**caractérisé en ce qu'**on transforme au moins un trifluorométhanesulfonate de formule générale (II),
(M^{a+}) [(OSO₂CF₃)⁻]ₐ (II)
dans laquelle M^{a+} représente un cation monovalent ou divalent et a = 1 ou 2,
dans une solution avec du bis(trifluorométhyl)imido-rubidium et on purifie et/ou isole le cas échéant le sel de type bis(trifluorométhyl)imido de formule générale (I) ainsi obtenu selon des procédés usuels.

2. Procédé selon la revendication 1, **caractérisé en ce que** M^{a+} représente un cation de sodium, de potassium, de césium, de cuivre ou d'argent et a = 1.

3. Procédé selon la revendication 1, **caractérisé en ce que** M^{a+} représente un cation de mercure, de cuivre, de zinc ou de cadmium et a = 2.

4. Procédé selon la revendication 1, **caractérisé en ce que** a = 1 et M^{a+} signifie un cation de formule générale (III),
[([R_{b}¹, R_{c}² R_{d}³ Rₑ⁴] Aₓ)_{y}Kt]⁺ (III)
où
Kt = N, P, As, Sb, S, Se,
A = N, P, P(O), O, S, S(O), SO₂, As, As(O), Sb, Sb(O),
R¹, R², R³ et R⁴ sont identiques ou différents et représentent,
H, halogène, alkyle CₙH₂ₙ₊₁ substitué et/ou non substitué, C₁₋₁₈-alcényle substitué et/ou non substitué et une ou plusieurs doubles liaisons, C₁₋₁₈-alcynyle substitué et/ou non substitué et une ou plusieurs triples liaisons, cycloalkyle CₘH₂ₘ₋₁ substitué et/ou non substitué, phényle monosubstitué ou polysubstitué et/ou non substitué, hétéroaryle substitué et/ou non substitué
avec
n = 1 - 18,
m = 3 - 7,
x = 0 ou 1,
y = 1 - 4, y = 1 pour x = 0,
où
b, c, d, e à chaque fois = 0 ou 1 avec b + c + d + e ≠ 0,
A peut être inclus en différentes positions dans R¹, R², R³ et/ou R⁴,
Kt peut être inclus dans un cycle ou un hétérocycle,
les groupes liés sur Kt peuvent être identiques ou différents.

5. Procédé selon l'une quelconque des revendications 1 à 4, **caractérisé en ce que** la transformation en un sel de type bis(trifluorométhyl)imido de formule générale (I) est réalisée à une température de -60 à +60°C, de préférence à une température de -50 à +50°C, de manière particulièrement préférée à une température de -45 à +30°C.

6. Procédé selon l'une quelconque des revendications 1 à 5, **caractérisé en ce que** la transformation en un sel de bis(trifluorométhyl)imido de formule générale (I) est réalisée dans un solvant organique, de préférence un solvant organique polaire.

7. Procédé selon la revendication 6, **caractérisé en ce qu'**on utilise, comme solvant, l'acétonitrile, le benzonitrile, le diméthoxyéthane et/ou le propionitrile ou un mélange d'acétonitrile, de benzonitrile, de diméthoxyéthane et/ou de propionitrile.

8. Procédé selon l'une quelconque des revendications 1 à 7, **caractérisé en ce que** le solvant contient ≤ 0,1% en poids, de préférence ≤ 0,01% en poids, de manière particulièrement préférée ≤ 0,005% en poids d'eau.

9. Procédé selon l'une quelconque des revendications 1 à 8, **caractérisé en ce que** le sel de trifluorométhanesulfonate de formule générale (II) ou le bis(trifluorométhyl)imido-rubidium sont utilisés avec un excès molaire ≤ 3% ou en des quantités équimolaires.

10. Procédé selon l'une quelconque des revendications 1 à 9, **caractérisé en ce que** le sel à base de bis(trifluorométhyl)imido de formule générale (I) est purifié par filtration, de préférence par filtration à une température de -90 à +30°C, de manière particulièrement préférée de -70 à +20°C et après élimination du solvant.

11. Procédé selon l'une quelconque des revendications 1 à 10, **caractérisé en ce que** le sel de type bis(trifluorométhyl)imido de formule générale (I) est purifié par extraction avec du dichlorométhane et/ou de l'hexane et/ou du diéthyléther.

12. Composés de formule générale (I)
M^{a+}[(N(CF₃)₂)⁻]ₐ (I)
où
M = Cd²⁺, Zn²⁺ ou Cu⁺.

13. Electrolyte contenant au moins un sel de formule générale (I) préparé selon la revendication 1.

14. Utilisation des électrolytes selon la revendication 13 dans des cellules électrochimiques.

15. Cellules électrochimiques, en particulier condensateurs et supercondensateurs, contenant un électrolyte selon la revendication 13.
